# EUROPEAN PATENT SPECIFICATION

(11) **EP 0 554 400 B1**
(45) Date of publication and mention of the grant of the patent: **23.07.1997**
(21) Application number: 92900484.4
(22) Date of filing: 18.11.1991
(51) Int. Cl.: C07D 215/48, C07D 295/13, C07C 311/46, C07C 311/47, C07C 317/50, C07C 323/60, C07C 275/14, C07C 275/16, C07C 275/24, C07C 275/26, C07K 5/02

(54) **RETROVIRAL PROTEASE INHIBITORS**
RETROVIRALE PROTEASE-INHIBITOREN
INHIBITEURS DE PROTEASES RETROVIRALES

(30) Priority: 19.11.1990 US 615210; 14.11.1991 US 789644
(43) Date of publication of application: 11.08.1993
(73) Proprietor: MONSANTO COMPANY, St. Louis Missouri 63167 (US)
(72) Inventor: DECRESCENZO, Gary, Anthony, St. Peters, MO 63376 (US); FRESKOS, John, Nicholas, Clayton, MO 63105 (US); GETMAN, Daniel, Paul, St. Louis, MO 63146 (US); LIN, Ko-Chung, St. Louis, MO 63146 (US); ROGIER, Donald, Joseph, Jr., St. Louis, MO 63114 (US); TALLEY, John, Jeffrey, Chesterfield, MO 63017 (US)
(74) Representative: Ernst, Hubert
(86) International application number: US9108582
(87) International publication number: WO9208698

(56) References cited:
- EP-A- 0 264 795
- FR-A- 2 620 451
- GB-A- 2 184 730
- GB-A- 2 200 115

## Description

### BACKGROUND OF THE INVENTION

### 1. Field of the Invention

The present invention relates to retroviral protease inhibitors and, more particularly, relates to novel compounds and a composition and method for inhibiting retroviral proteases. This invention, in particular, relates to urea-containing hydroxyethylamine protease inhibitor compounds, a composition and method for inhibiting retroviral proteases such as human immunodeficiency virus (HIV) protease and for treating a retroviral infection, e.g., an HIV infection. The subject invention also relates to processes for making such compounds as well as to intermediates useful in such processes.

### 2. Related Art

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral replication proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP 0 346 847; EP 0 342,541; Roberts et al, "Rational Design of Peptide-Based Proteinase Inhibitors "Science, 248, 358 (1990); and Erickson et al, "Design Activity, and 2.8Å crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP 0 264 795; G.B. 2,200,115 and U.S. SIR H725. Of these, G.B. 2,200,115, GB 2,209,752, EP 0 264,795, U.S. SIR H725 and U.S. 4,599,198 disclose urea-containing hydroxyethylamine renin inhibitors. However, it is known that, although renin and HIV proteases are both classified as aspartyl proteases, compounds which are effective renin inhibitors generally cannot be predicted to be effective HIV protease inhibitors.

Compounds which are useful for the treatment of retroviral infections are also described in FR-A-2 620 451. This document discloses intermediates which are structurally related to the compounds of the present invention. However, no antiviral activity was reported or suggested for such intermediates.

### BRIEF DESCRIPTION OF THE INVENTION

The present invention is directed to virus inhibiting compounds and compositions. More particularly, the present invention is directed to retroviral protease inhibiting compounds and compositions, to a method of inhibiting retroviral proteases, to processes for preparing the compounds and to intermediates useful in such processes. The subject compounds are characterized as urea-containing hydroxyethylamine inhibitor compounds.

### DETAILED DESCRIPTION OF THE INVENTION

In accordance with the present invention, there is provided a retroviral protease inhibiting compound of the formula: 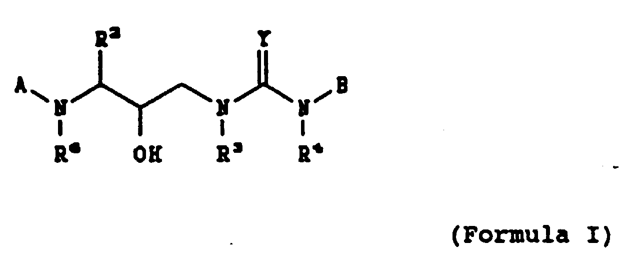 or a pharmaceutically acceptable salt, thereof
wherein A is a alkanoyl, aralkanoyl, aroyl, aryloxyalkanoyl, heteroaroyl, alkoxycarbonyl, aralkoxycarbonyl, aryloxycarbonyl, cycloalkylalkoxycarbonyl, cycloalkylcarbonyl, heteroaralkoxycarbonyl, heterocyclylcarbonyl, heterocyclyloxycarbonyl, alkylaminocarbonyl, aralkylaminocarbonyl or dialkylaminoalkanoyl radical;
B represents R⁵ and radicals represented by the formula 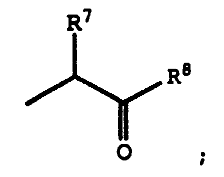
Y is O or S
R² is an alkyl, aryl, cycloalkyl, cycloalkylalkyl or aralkyl radical, which radical is optionally substituted with -OR⁹, -SR⁹, or halogen radical, wherein R⁹ is a hydrogen or alkyl radical;
R³ is a hydrogen, alkyl, alkenyl, hydroxyalkyl, cycloalkyl, cycloalkylalkyl, heterocycloalkylalkyl, aryl, aralkyl or heteroaralkyl radical;
R⁴ is a hydrogen, alkyl, alkenyl, hydroxyalkyl, cycloalkyl, cycloalkylalkyl, heterocycloalkylalkyl, aryl, aralkyl or heteroaralkyl radical; or R⁴ and B together with the nitrogen atom to which they are bonded form a heterocycloalkyl radical;
R⁶ is a hydrogen or alkyl radical;
R⁷ is an alkyl radical, or the side chain of an amino acid selected from valine, isoleucine, glycine, alanine, allo-isoleucine, asparagine, leucine, glutamine or t-butylglycine; and
R⁸ is an amide derivative of an amino acid; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with an alkyl, alkoxy, halogen, hydroxy or amino radical; heterocycloalkyl, alone or in combination, is a saturated or partially unsaturated monocyclic, bicyclic or tricyclic heterocycle, wherein at least one ring member is a nitrogen, oxygen or sulfur atom and which is unsubstituted or substituted on a substitutable carbon atom with halogen, alkyl, alkoxy or oxo, on a ring secondary nitrogen atom with alkyl, aralkoxycarbonyl, alkanoyl, phenyl or phenylalkyl, or on a ring tertiary nitrogen atom by oxide; and heteroaryl, alone or in combination, is an aromatic monocyclic, bicyclic or tricyclic heterocycloalkyl radical.

A preferred class of compounds are those represented by the formula: 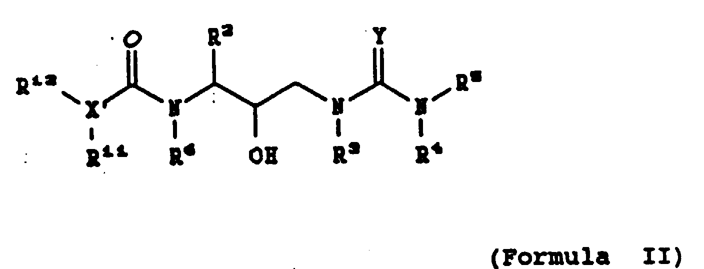 or a pharmaceutically acceptable salt, thereof wherein
- X': represents O, C(R¹⁷) where R¹⁷ represents hydrogen and alkyl radicals, and N;
- Y: represent O,S;
- R⁴, R⁵, R¹¹ and R¹²: independently represent hydrogen and radicals as defined by R^{3'} or R⁴ and R⁵, and/or R¹¹ and R¹² together with the nitrogen atom to which they are bonded represent heterocycloalkyl and heteroaryl radicals, or R¹¹ and R¹² together with a carbon atom to which they are attached represent cycloalkyl and aryl radicals;

As utilized herein, the term "alkyl", alone or in combination, means a straight-chain or branched-chain alkyl radical containing from 1 to 8 carbon atoms. Examples of such radicals include methyl, ethyl, n-propyl, isopropyl, n-butyl, isobutyl, sec-butyl, tert-butyl, pentyl, isoamyl, hexyl, octyl and the like. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert-butoxy and the like. The term "cycloalkyl" means an alkyl radical which contains from 3 to 8 carbon atoms and is cyclic. The term "cycloalkylalkyl" means an alkyl radical as defined above which is substituted by a cycloalkyl radical containing from 3 to 8, preferably from 3 to 6, carbon atoms. Examples of such cycloalkyl radicals include cyclopropyl, cyclobutyl, cyclopentyl, cyclohexyl and the like. The term "aryl", alone or in combination, means a phenyl or naphthyl radical which optionally carries one or more substituents selected from alkyl, alkoxy, halogen, hydroxy, amino and the like, such as phenyl, p-tolyl, 4-methoxyphenyl, 4-(tert-butoxy)phenyl, 4-fluorophenyl, 4-chlorophenyl, 4-hydroxyphenyl, 1-naphthyl, 2-naphthyl, and the like. The term "aralkyl", alone or in combination, means an alkyl radical as defined above in which one hydrogen atom is replaced by an aryl radical as defined above, such as benzyl, 2-phenylethyl and the like. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "aryloxy" means a radical of the formula aryl-o- in which the term aryl has the significance given above. The term "alkanoyl", alone or in combination, means an acyl radical derived from an alkanecarboxylic acid, examples of which include acetyl, propionyl, butyryl, valeryl, 4-methylvaleryl, and the like. The term "cycloalkylcarbonyl" means an acyl group derived from a monocyclic or bridged cycloalkanecarboxylic acid such as cyclopropanecarbonyl, cyclohexanecarbonyl, adamantanecarbonyl, and the like, or from a benz-fused monocyclic cycloalkanecarboxylic acid which is optionally substituted by, for example, alkanoylamino, such as 1,2,3,4-tetrahydro-2-naphthoyl,2-acetamido-1,2,3,4-tetrahydro-2-naphthoyl. The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydroinnamoyl,4-methoxyhydrocinnamoyl, and the like. The term "aroyl" means an acyl radical derived from an aromatic carboxylic acid. Examples of such radicals include aromatic carboxylic acids, an optionally substituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 6-carboxy-2 naphthoyl, 6-(benzyloxycarbonyl)-2-naphthoyl, 3-benzyloxy-2-naphthoyl, 3-hydroxy-2-naphthoyl, 3-(benzyloxyformamido)-2-naphthoyl, and the like. The heterocyclyl or heterocycloalkyl portion of a heterocyclylcarbonyl, heterocyclyloxycarbonyl, heterocyclylalkoxycarbonyl, or heterocyclyalkyl group or the like is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy, oxo, and the like, and/or on a secondary nitrogen atom (i.e., -NH-) by alkyl, aralkoxycarbonyl, alkanoyl, phenyl or phenylalkyl or on a tertiary nitrogen atom (i.e. = N-) by oxido and which is attached via a carbon atom. The heteroaryl portion of a heteroaroyl, heteroaryloxycarbonyl, or a heteroaralkoxy carbonyl group or the like is an aromatic monocyclic, bicyclic, or tricyclic heterocycle which contains the hetero atoms and is optionally substituted as defined above with respect to the definition of heterocyclyl. Examples of such heterocyclyl and heteroaryl groups are pyrrolidinyl, piperidinyl, piperazinyl, morpholinyl, thiamorpholinyl, pyrrolyl, imidazolyl (e.g., imidazol 4-yl, 1-benzyloxycarbonylimidazol-4-yl, etc.), pyrazolyl, pyridyl, pyrazinyl, pyrimidinyl, furyl, thienyl, triazolyl, oxazolyl, thiazolyl, indolyl (e.g., 2-indolyl, etc.), quinolinyl, (e.g., 2-quinolinyl, 3-quinolinyl, 1-oxido-2-quinolinyl, etc.), isoquinolinyl (e.g., 1-isoquinolinyl, 3-isoquinolinyl, etc.), tetrahydroquinolinyl (e.g., 1,2,3,4-tetrahydro-2-quinolyl, etc.), 1,2,3,4-tetrahydroisoquinolinyl (e.g., 1,2,3,4-tetrahydro-1-oxo-isoquinolinyl, etc.), quinoxalinyl, β-carbolinyl, 2-benzofurancarbonyl, 2-benzimidazolyl and the like. The term "cycloalkylalkoxycarbonyl" means an acyl group derived from a cycloalkylalkoxycarboxylic acid of the formula cycloalkylalkyl-O-COOH wherein cycloalkylalkyl has the significance given above. The term "aryloxyalkanoyl" means an acyl radical of the formula aryl-O-alkanoyl wherein aryl and alkanoyl have the significance given above. The term "heterocyclyloxycarbonyl" means an acyl group derived from heterocyclyl-O-COOH wherein heterocyclyl is as defined above. The term "heterocyclylalkanoyl" is an acyl radical derived from a heterocyclyl-substituted alkane carboxylic acid wherein heterocyclyl has the significance given above. The term "heterocyclylalkoxycarbonyl" means an acyl radical derived from a heterocyclyl-substituted alkane-O-COOH wherein heterocyclyl has the significance given above. The term "heteroaryloxycarbonyl" means an acyl radical derived from a carboxylic acid represented by heteroaryl-O-COOH wherein heteroaryl has the significance given above. The term "aminoalkanoyl" means an acyl group derived from an amino-substituted alkanecarboxylic acid wherein the amino group can be a primary, secondary or tertiary amino group containing substituents selected from hydrogen, and alkyl, aryl, aralkyl, cycloalkyl, cycloalkylalkyl radicals and the like. The term "halogen" means fluorine, chlorine, bromine or iodine. The term "leaving group" generally refers to groups readily displaceable by a nucleophile, such as an amine, a thiol or an alcohol nucleophile. Such leaving groups are well known and include carboxylates, N-hydroxysuccinimide, N-hydroxybenzotriazole, halides, triflates, tosylates -OR and -SR and the like. Preferred leaving groups are indicated herein where appropriate.

Procedures for preparing the compounds of Formula-I are set forth below. It should be noted that the general procedure is shown as it relates to preparation of compounds having the specified stereochemistry, for example, wherein the stereochemistry about the hydroxyl group is designated as (R). However, such procedures are generally applicable, to those compounds of opposite configuration, e.g., where the stereochemistry about the hydroxyl group is (S).

### Preparation of Compounds of Formula I

The compounds of the present invention represented by Formula I above can be prepared utilizing the following general procedure. An N-protected chloroketone derivative of an amino acid having the formula: 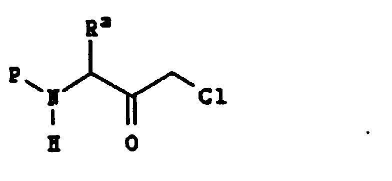 wherein P represents an amino protecting group, and R² is as defined above, is reduced to the corresponding alcohol utilizing an appropriate reducing agent. Suitable amino protecting groups are well known in the art and include carbobenzoxy, butyryl, t-butoxycarbonyl, acetyl, benzoyl and the like. A preferred amino protecting group is carbobenzoxy. A preferred N-protected chloroketone is N-benzyloxycarbonyl-L-phenylalanine chloromethyl ketone. A preferred reducing agent is sodium borohydride. The reduction reaction is conducted at a temperature of from -10°C to 25°C, preferably at 0°C, in a suitable solvent system such as, for example, tetrahydrofuran, and the like. The N-protected chloroketones are commercially available from Bachem, Inc., Torrance, California. Alternatively, the chloroketones can be prepared by the procedure set forth in S. J. Fittkau, J. Prakt. Chem., 315, 1037 (1973), and subsequently N-protected utilizing procedures which are well known in the art.

The resulting alcohol is then reacted, preferably at room temperature, with a suitable base in a suitable solvent system to produce an N-protected amino epoxide of the formula: 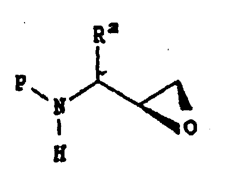 wherein P and R² are as defined above. Suitable solvent systems for preparing the amino epoxide include ethanol, methanol, isopropanol, tetrahydrofuran, dioxane, and the like including mixtures thereof. Suitable bases for producing the epoxide from the reduced chloroketone include potassium hydroxide, sodium hydroxide, potassium t-butoxide, DBU and the like. A preferred base is potassium hydroxide.

The amino epoxide is then reacted, in a suitable solvent system, with an equal amount, or preferably an excess of, a desired amine of the formula:

R³NH₂

wherein R³ is hydrogen or is as defined above. The reaction can be conducted over a wide range of temperatures, e.g., from 10°C to 100°C, but is preferably, but not necessarily, conducted at a temperature at which the solvent begins to reflux. Suitable solvent systems include those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. The resulting product is a 3-(N-protected amino)-3-(R²)-1-(NHR³)-propan-2-ol derivative (hereinafter referred to as an amino alcohol) can be represented by the formula: 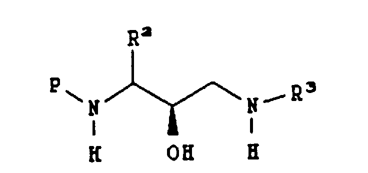 wherein P, R² and R³ are as described above.

For producing compounds of Formula I wherein R⁵ is hydrogen, the resulting amino alcohol described above is then reacted, in a suitable solvent system, with an isocyanate of the formula R⁴NCO wherein R⁴ is as defined above. Where Y in Formula I above is sulfur, the resulting amino alcohol is reacted with an isothiocyanate of the formula R⁴NCS under similar conditions. Suitable solvent systems include tetrahydrofuran, methylene chloride, and the like and mixtures thereof. The resulting product is a urea derivative of the amino alcohol or the corresponding sulfur analog thereof and can be represented by the formula: 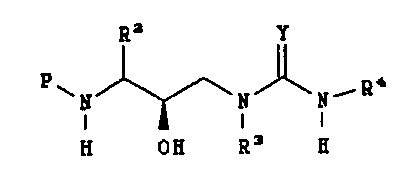 wherein P, Y, R², R³ and R⁴ are as defined above. The isocyanates of the formula R⁴NCO can be prepared by the reaction of an amine (R³NH₂) with phosgene, triphosgene, carbodiimidazole, or carbonate ((RO)₂CO) under conditions well-known in the art. The isothiocyanates of the formula R4NCS can be prepared by similar procedures, e.g., reaction of the amine with thiophosgene, which are also well known in the art. In addition, the isocyanates and isothiocyanates are commercially available from Aldrich Chemical Company.

For preparing compounds of Formula I wherein R⁵ is other than hydrogen, the resulting amino alcohol described above is then reacted, in a suitable solvent system, with a compound represented by the formula: 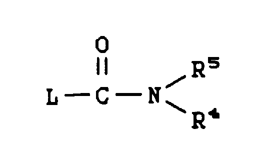 wherein R⁴ and R⁵ are as described above and L represents a leaving group such as a halide, e.g., chloride, imidazole radical, the radical p-NO₂-(C₆H₄)-O-, and the like. A preferred compound represented by this formula is a carbamoyl chloride. The corresponding sulfur analogs can be utilized where Y of Formula I is S.

The urea derivative of the amino alcohol and the corresponding sulfur analog can be represented by the formula:

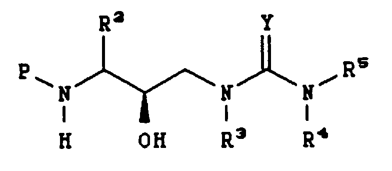

Following preparation of the urea derivative, or corresponding analogs wherein Y is S, the amino protecting group P can be removed under conditions which will not affect the remaining portion of the molecule. These methods are well known in the art and include acid hydrolysis, hydrogenolysis and the like. A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative.

The amine salt derivative can then be reacted with a carboxylate represented by the formula: 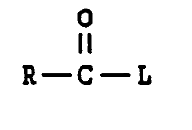 wherein R is as defined above and L is an appropriate leaving group such as a halide. A solution of the free amine (or amine acetate salt) and 1.0 equivalent of the carboxylate are mixed in an appropriate solvent system and optionally treated with up to five equivalents of a base such as, for example, N-methylmorpholine, at about room temperature. Appropriate solvent systems include tetrahydrofuran, methylene chloride or N,N-dimethylformamide, and the like, including mixtures thereof.

### Preparation of Compounds of Formula II

The compounds of the present invention represented by Formula II can be prepared by the following generalized procedure. Following the procedure for preparing compounds of Formula I above, the urea derivative, or corresponding sulfur analog thereof, of an amino alcohol is prepared having the formula: 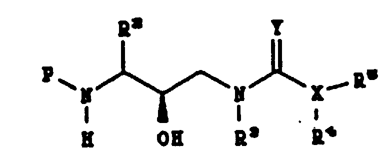 wherein P, Y, R², R³, R⁴, and R⁵ are as defined above. The amino protecting group is then removed following the procedure as outlined above for preparing compounds of Formula I and reacted again with either an isocyanate of the formula R¹¹NCO, an isothiocyanate of the formula R¹¹NCS or a compound of the formula: 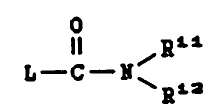 wherein R¹¹, R¹² and L are as defined above to form the bis-urea derivative or C or O analog thereof represented by the formula: 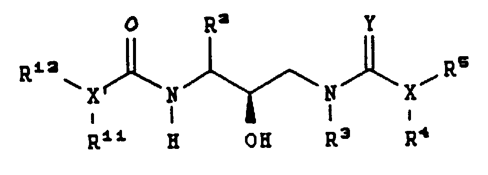 wherein X', Y, R², R³, R⁴, R⁵, R¹¹ and R¹² are as defined above.

It is contemplated that for preparing compounds of the Formulas having R⁶, the compounds can be prepared following the procedure set forth above and the procedure referred to in the art as reductive amination. Thus, a sodium cyanoborohydride and an appropriate aldehyde R⁶C(O)H or ketone R⁶C(O)R⁶ can be reacted with the urea derivative compound or appropriate analog at room temperature in order to reductively aminate any of the compounds of Formulas I-IV. It is also contemplated that where R³ of the amino alcohol intermediate is hydrogen, the inhibitor compounds can be prepared through reductive amination of the final product of the reaction between the amino alcohol and the amine or at any other stage of the synthesis for preparing the inhibitor compounds.

Contemplated equivalents of the general formulas set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

All reagents were used as received without purification. All proton and carbon NMR spectra were obtained on either a Varian VXR-300 or VXR-400 nuclear magnetic resonance spectrometer.

### Example 1

Exemplary compounds of the present invention were prepared by the following methods.

### METHOD A - Urea formation from 3(S)-benzyloxycarbonylamino-2(R)hydroxy 4-phenyl butyl amine and an amino acid or peptide utilizing carbodiimidazole

Part A
   A Fisher-Porter bottle was charged with 3(S)-(benzyloxycarbonyl)amino-1,2(S)epoxy-phenylbutane (520 mg, 1.75 mmol) and 20 ml of isopropyl alcohol.
   Anhydrous ammonia was then bubbled into the solution at 0°C until the solution was saturated. The bottle was capped and the solution warmed to 50°C for 16 hours. The solvent was removed in vacuo and a white solid was isolated, 460 mg, 84% 3(S)-benzyloxycarbonylamino-2(R)-hydroxy-4-phenylbutyl amine, which was used directly in the next step.
Part B
   L-leucine methyl ester hydrochloride (265.9 mg, 1.465 mmol) was dissolved in 20 ml of chloroform at 50°C. To this solution was added carbonyldiimidazole (237.6 mg, 1.465 mmol). The crude amino alcohol from Part A was dissolved in chloroform and added to the reaction mixture. The mixture was maintained at 50°C for 16 hours. The reaction mixture was cooled to room temperature and poured into a separatory funnel and washed with 1N KHSO₄, saturated aqueous NaHCO₃ saturated aqueous NaCl, the chloroform layer was dried over anhydrous magnesium sulfate, filtered and concentrated in vacuo to give a white solid. The solid was recrystallized from ethyl acetate to give 503 mg, 71% of pure [[[3(S)-3[[1,1-dimethylethoxycarbonyl]]amino 2(S)-hydroxy-4-phenylbutyl][amino carbonyl]]]-L-leucine methyl ester. TLC SiO₂ 1:1 hexane: EtOAC R_{f} = 0.52, mp 156.5-157.5°C.

### METHOD B - Preparation Utilizing Peptide Coupling

Part A
   L-Leucine methyl ester hydrochloride (527 mg, 2.9 mmol), carbonyldiimidazole (470 mg, 29 mmol) and 50 ml of chloroform were warmed to 50°C for 1.5 hours and then treated with 3(S)-1,1-dimethylethoxycarbonylamino- 2(S)-hydroxy 4-phenylbutyl methyl amine (852 mg, 2.9 mmol) followed by triethylamine (404 mg, 4.0 mmol). The mixture was maintained at 50°C for an additional 2 hours and then the reaction mixture was poured into a separatory funnel. The chloroform solution was washed with saturated aqueous NaHCO₃, twice with 1N aq. KHSO₄, once with water, and once with brine. The organic phase was dried over anhyd. MgSO₄, filtered and concentrated to give 1.38 g of an oil. The oil was chromatographed on SiO₂ eluting with hexanes/ethyl acetate to give N-[[[3(S)-3[[1,1-dimethylethoxycarbonyl]]amino-2(S)-hydroxy-4-phenylbutyl][methylaminocarbonyl]]]-L-leucine methyl ester as a glassy foam, 870 mg, 65%, TLC on SiO₂ eluting with 1:1 hexane : ethyl acetate showed R_{f}= 0.35.
Part B
   The purified product from Part A (580 mg, 1.24 mmol) was treated with a solution of lithium hydroxide (65 mg, 1.5 mmol, 1.2 eq) in water (12 ml) and methanol (15 ml). The reaction was stirred at room temperature for 13 hours. The solution was concentrated in vacuo and diluted with water, acidified to pH=1 with 1N KHSO₄, filtered and concentrated in vacuo to give 560 mg of N-[[[3(S)-3[[1,1-dimethylethoxycarbonyl]]amino-2(S)-hydroxy-4-phenylbutyl][methylamino carbonyl]]]-L-leucine as a glassy foam, mp 145-146°C 100%.
Part C
   The product from Part B (219 mg, 0.486 mmol), 1-hydroxybenzotriazole (74.3 mg, 0.486 mmol), 1-(3-dimethylaminopropyl)-3-ethylcarbodiimide hydrochloride (93.1 mg, 0.486 mmol), and L-phenylalanine methyl ester hydrochloride (105 mg, 0.486 mmol) were dissolved in 10 ml of chloroform. The solution was cooled to 0°C and treated with N-methyl morpholine (54 mg, 0.534 mmol) via syringe. The reaction was maintained at 0°C for 1 hour and at room temperature for an additional hour. The reaction was poured into a separatory funnel and diluted with chloroform and washed twice with 1N aqueous KHSO₄, once with H₂O, twice with saturated aqueous NaHCO₃, once with brine. dried over anhydrous MqSO₄, filtered and concentrated in vacuo to give a white solid. The solid was recrystallized from ethyl acetate/hexane to give 160 mg, 54% of pure N[[[3(S)-3[[1,1-dimethylethoxycarbonyl]]amino-2(S)-hydroxy-4-phenylbutyl][methylaminocarbonyl]]]-L-leucyl-L-phenylalanine methyl ester, mp 150-151°C. TLC SiO₂ 2:1 ethyl acetate:hexane R_{f}=0.50.
Part D
   A sample of the product from Part C (48.6 mg, 0.79 mmol) was dissolved in anhydrous methanol and treated in with anhydrous ammonia at 0°C until the solution was saturated. The reaction was allowed to stand undisturbed for 96 hours and then concentrated in vacuo. The crude solid was taken up in ether with a small amount of isopropyl alcohol and allowed to stand undisturbed whereupon crystals of pure N-[[3(S)-3[[1,1-dimethylethoxycarbonyl]]amino-2(S)-hydroxy-4-phenylbutyl][methylaminocarbonyl][-L-leucyl-L-phenylalanyl]]] amide 21.4 mg, 45%, mp 120-122°C TLC SiO₂ 100% ethyl acetate R_{f}=0.33.

### METHOD C - Synthesis Via Dipeptide Coupling

Part A
   L-Leucyl-L-phenylalanine methyl ester hydrochloride. A solution of Boc-L-Leucyl-L-phenylalanine methyl ester (2.01g, 5.13 mmol) in 10 ml of 4N HCl in dioxane was stirred at room temperature for 45m whereupon the entire solution solidified. The solid was crushed and slurried with anhydrous ether and isolated by filtration on a Buchner funnel. The solid was dried in vacuo to give 1.65g, 97%, of L-leucyl-L-phenylalanine methyl ester hydrochloride suitable for subsequent reaction.
Part B
   A solution of 3(S)-1,1-dimethylethoxycarbonyl, amino 1,2(S)-epoxy 4-phenyl butane (1.30g, 4.94 mmol) in 40 ml of isopropyl alcohol was treated with benzyl amine (529 mg, 4.94 mmol) at 50°C for 16 hours. The solution was concentrated in vacuo to give an oil that was triturated with n-hexane to give N-[[3(S)-3[1,1-dimethylethoxycarbonyl]amino-2(S)-hydroxy-4-phenylbutyl]]N-benzylamine, a white solid, 1.36 g, 74% mp 87.5-89.5°C.
Part C
   A solution of L-leucyl L-phenylalanine methyl ester hydrochloride (172.1 mg, 0.524 mmol), in 5 ml of chloroform was treated with carbonyldiimidazole (89.2 mg, 0.55 mmol, 1.05 equiv.). The solution was cooled with an ice bath and treated with N-methyl morpholine (53 mg, 0.524 mmol) and then with imidazole (35.6 mg, 0.524 mmol). The solution was maintained at 0°C for 0.5 hour and then treated with the amino alcohol from Part B (193.8 mg, 0.524 mmol). The solution was allowed to warm to room temperature and maintained at that temperature for 3 hours. The reaction mixture was poured into a separatory funnel, diluted with additional chloroform and washed twice with 1N KHSO₄, twice with saturated aqueous NaHCO₃, once with brine, dried over anhydrous magnesium sulfate, filtered and concentrated in vacuo. The crude product was purified by radial chromatography on SiO₂ eluting with hexanes/ethyl acetate to give pure N-[[[3(S)-3-[[1,1-dimethylethoxycarbonyl]]amino-2(S)-hydroxy-4-phenylbutyl][benzylamino carbonyl]]]-L-leucyl-L-phenylalanine]]] methyl ester as a white solid 226.8 mg, 63%, mp 69.5-70.5°C. TLC on SiO₂ 1:1 hexanes : EtOAc R_{f} = 0.60.
Part D
   A sample of the purified material from Part C (59.1 mg, 0.85 mmol) in 3 ml of methanol was cooled to 0°C and treated with anhydrous ammonia until the solution was saturated. The mixture was set aside at room temperature for 24 hours and then concentrated in vacuo to give a white solid. This material was recrystallized from hexanes/ethyl acetate to give pure N-[[[3(S)-3-[[1,1-dimethylethoxycarbonyl]]amino-2(S)-hydroxy-4-phenylbutyl][benzylamino carbonyl]]]-L-leucyl-L-phenylalanyl amide 39.9 mg 69%, mp 137-138°C.

### METHOD D - Synthesis via Curtius Rearrangement

Part A
   A solution consisting of 4(S)1,1-dimethylethoxycarbonylamino-3(S)-hydroxy-5-phenyl pentanoic acid (800 mg, 2.59 mmol), imidazole (847 mg, 12.43 mmol), tert-butyldimethylsilyl chloride (937 mg, 6.22 mmol) in 10 ml of dry dimethylformamide was stirred at room temperature for 24 hours. The solvent was removed in vacuo and the residue dissolved in ethyl acetate; the solution was washed with water, 5% aqueous citric acid, water and brine. The ethyl acetate solution was dried over anhydrous MgSO₄, filtered and concentrated in vacuo to give a thick oil that was dissolved in 40 ml of methanol. The methanolic solution was then treated with 7 ml of 10% aqueous K₂CO₃. After stirring for an hour the solution was concentrated in vacuo and the residue dissolved in water. The aqueous phase was acidified to pH=1 with 1N KHSO₄ and then extracted three times with ether. The combined ethereal solution was washed with brine, dried over anhydrous MgSO₄, filtered and concentrated in vacuo to give 1.00 g, 98% of 4(S)-1,1-dimethylethoxycarbonylamino-3(S)tert-butyldimethylsilyloxy-5-pbenylpentanoic acid, as a white solid. The solid was recrystallized from n-hexane to provide 803 mg, 79% of pure acid, mp 148-149°C.
Part B
   A solution of the product from Part A (300 mg, 0.76 mmol) and triethylamine (153 mg, 1.52 mmol, 2.0 eq) was dissolved in 30 ml of toluene and warmed to 90°C. To this stirring solution was added diphenylphosphorylazide (219 mg, 0.80 mmol, 1.05 eq) via syringe. After 1.5 hours at 90°C the solution was treated with L-leucyl-L-phenylalanine methyl ester hydrochloride (251 mg, 0.80 mmol). The solution was allowed to stir at 45°C for 16 hours and then the solution was cooled to room temperature and poured into a separatory funnel. The solution was washed with water, twice with 1N KHSO₄, water, sat. aq. NaHCO₃, & brine. The solution was then dried over anhydrous MgSO₄, filtered and the material was purified by radial chromatography on SiO₂ eluting with hexanes/ethyl acetate to provide 270 mg, 50% of pure urea N-[[[3(S)-3-[[1,1-dimethylethoxycarbonyl]amino-2(S)-tert-butyldimethylsilyloxy,-4-phenylbutyl][amino carbonyl]]]-L-leucyl-L-phenylalanine methyl ester, mp 146-147°C.
Part C
   A solution of the product from Part B (250 mg, 0.35 mmol) in tetrahydrofuran (5 ml) was treated with a 1N solution of tetra-N-butylammonium fluoride (0.7 ml, 0.7 mmol, 2 eq). The solution was stirred for 1 hour at room temperature and then concentrated in vacuo. The residue was dissolved in ethyl acetate and washed four times with water, brine, dried over anhydrous MgSO₄, filtered and concentrated in vacuo to give N-[[[3(S)-3-[[1,1-dimethylethoxycarbonyl]]amino-2(S)-hydroxy-4-phenylbutyl][amino carbonyl]]]-L-leucyl-L-phenylalanine methyl ester as a white solid 200 mg, 96%, mp 180-182°C, TLC on SiO₂ 100% EtAc R_{f} = 0.64.
Part D
   The product from Part C (20.2 mg, 0.34 mmol) was dissolved in 1 ml of methanol and cooled to 0°C. The solution was saturated with anhyd. ammonia and then kept at room temperature for 48 hours. The solvent was removed in vacuo and was a white solid obtained and was recrystallized from methanol to give 19.5 mg, 97% of pure N-[[[3(S)-3[[1,1-dimethylethoxycarbonyl]]amino-2(S)-hydroxy-4-phenylbutyl][amino carbonyl]]]-L-leucyl-L-phenylalanyl amide mp 187-188°C (dec). TLC on SiO₂ 100% EtOAc R_{f} = 0.29.

### EXAMPLE 2

Following the procedures set forth in Example 46, the compounds set forth in Tables 1 and 2 were prepared.

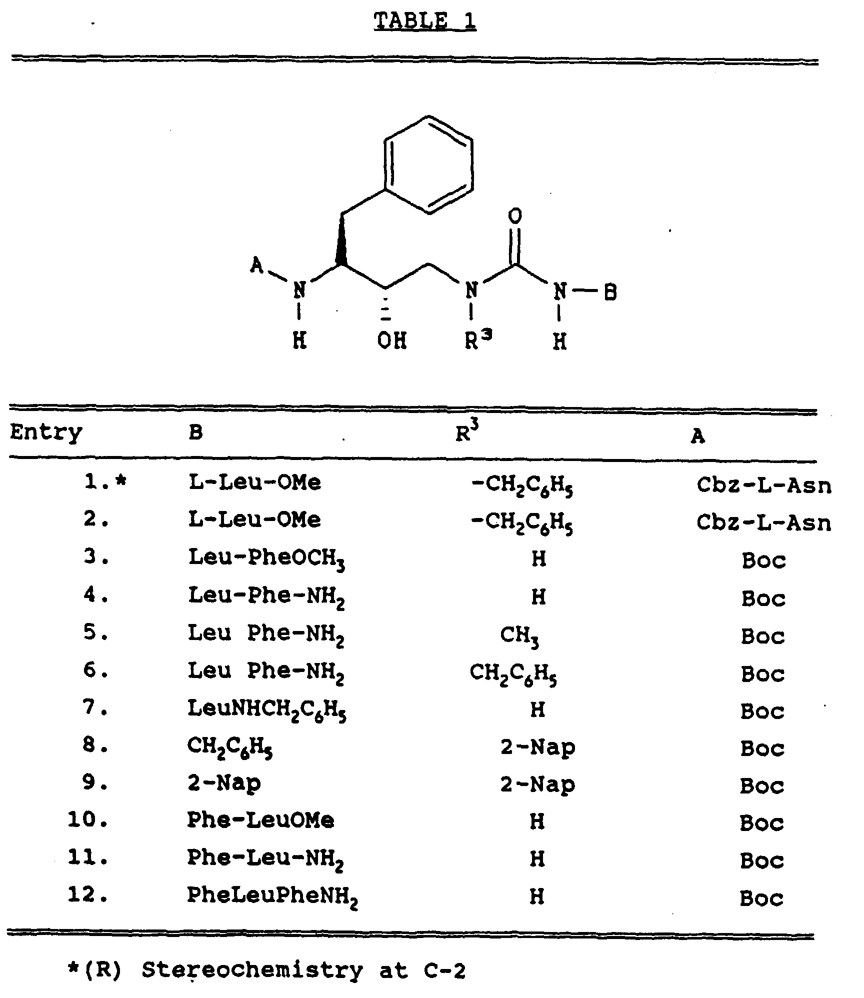

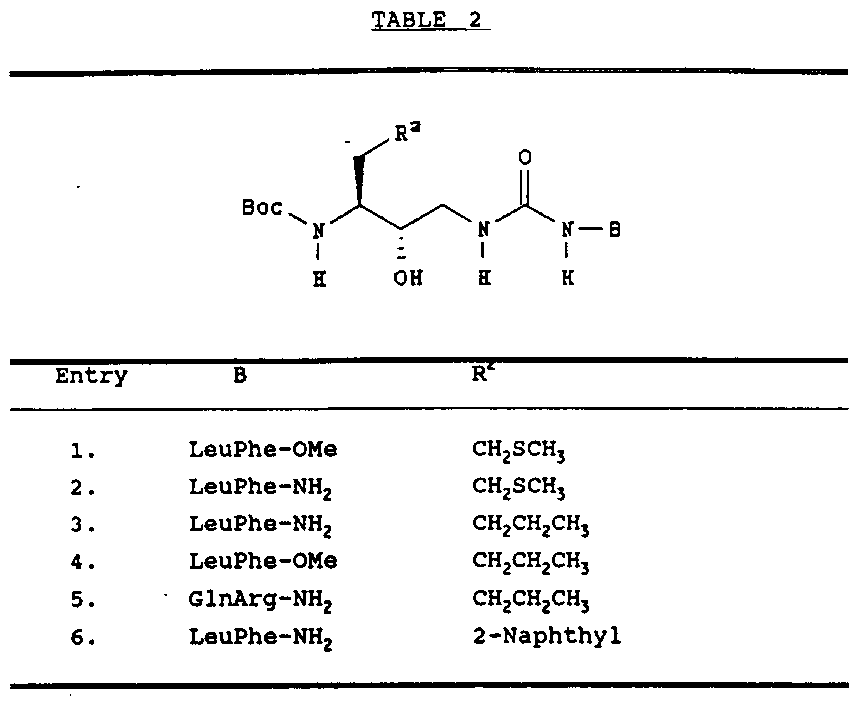

### Example 3

Following the procedures set forth above in Example 1, the compounds set forth in Tables 3 and 4 were prepared

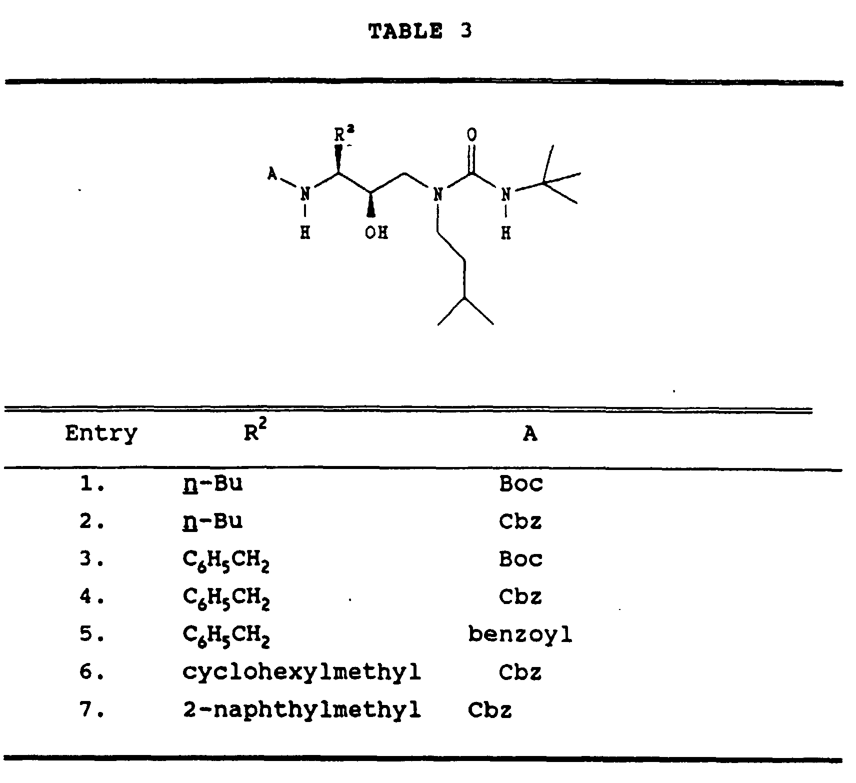

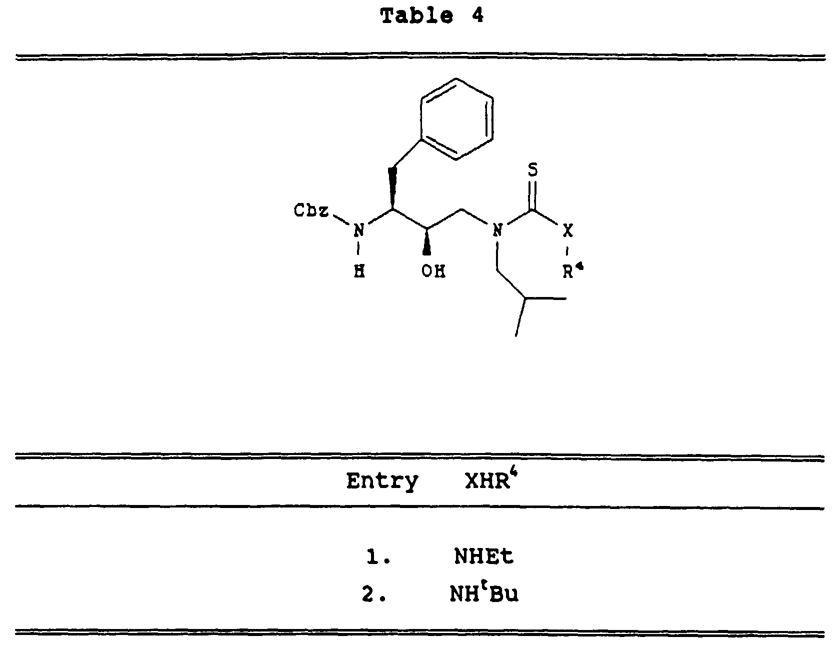

### EXAMPLE 4

The compounds of Table 5 were prepared according to the generalized procedures set forth above in Example 1.

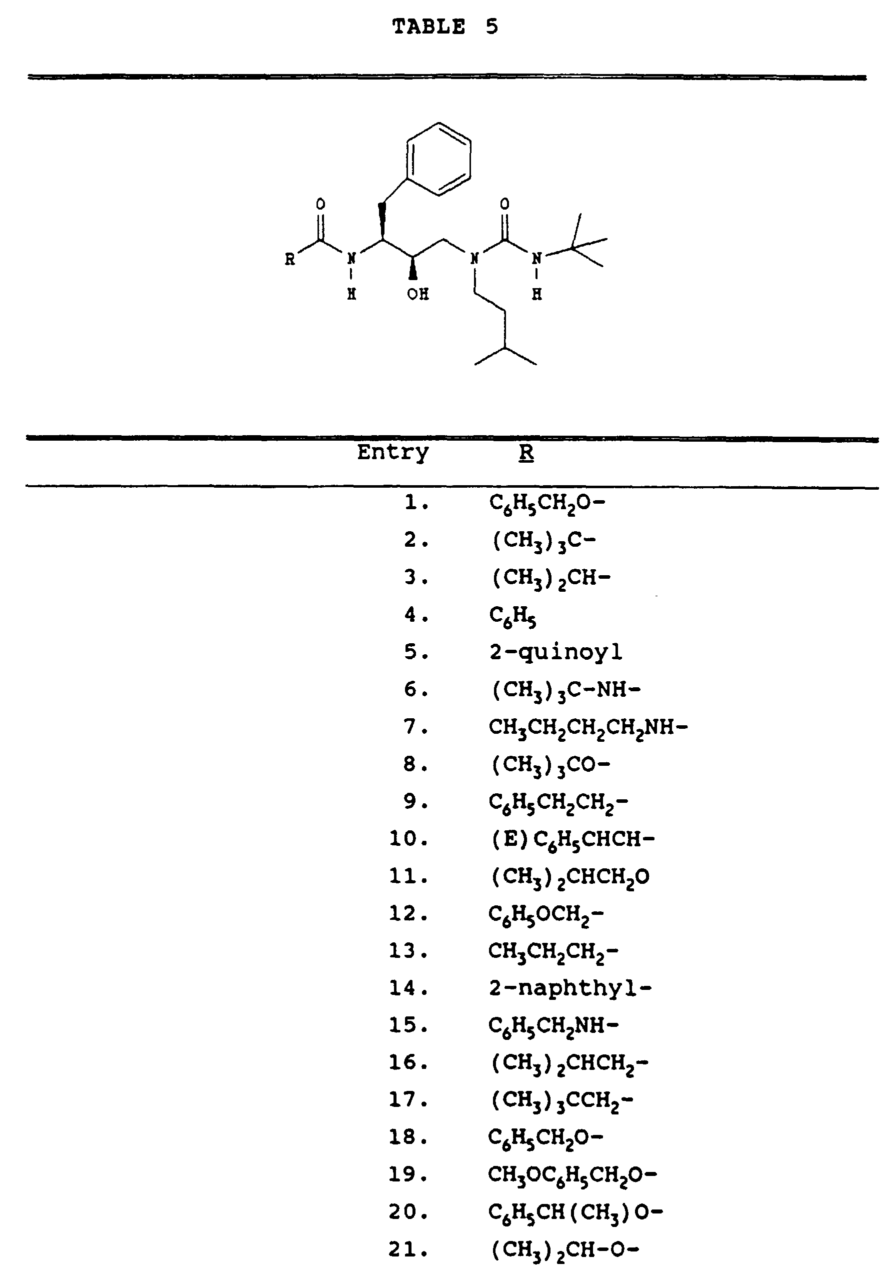

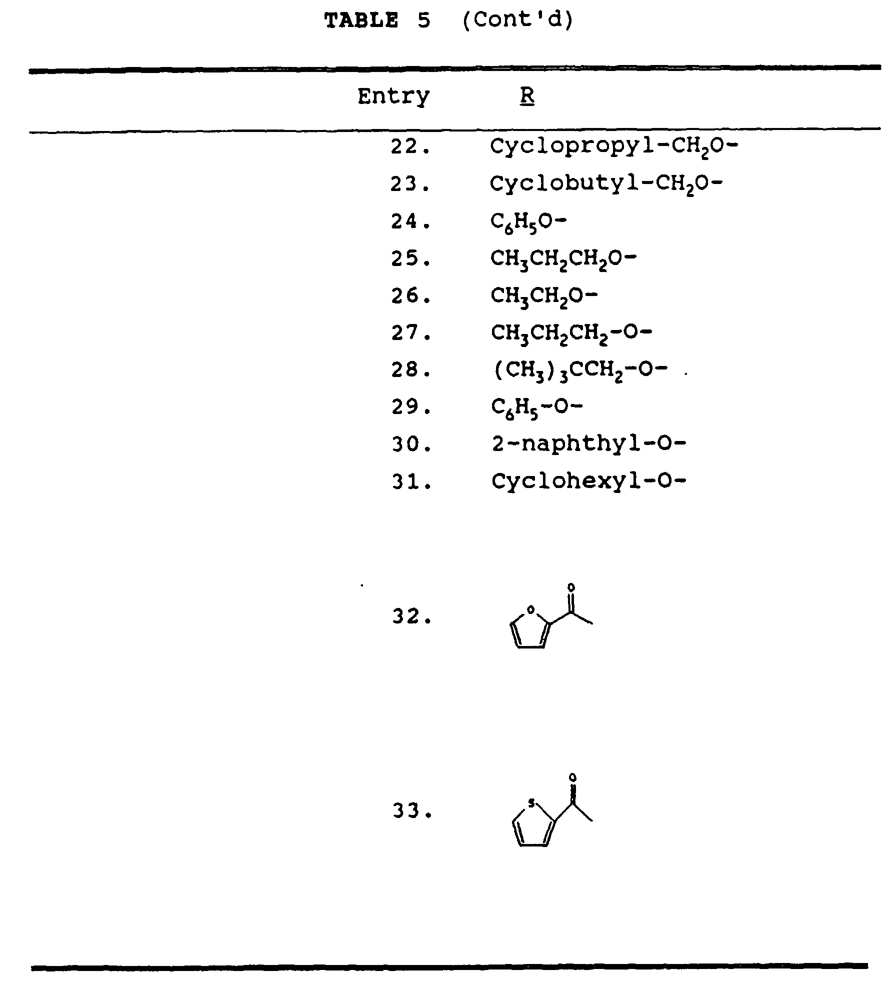

### EXAMPLE 5

A bis-t-butyl urea compound of the formula 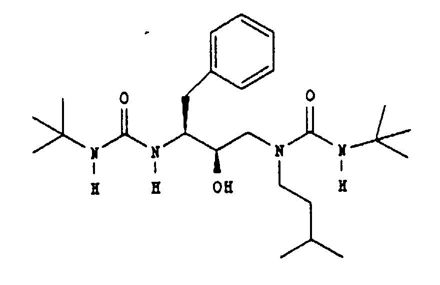 was prepared according to the following procedure.
Part A:
   A solution of 3(S)-(benzyloxycarbonyl)-1,2-(S)epoxy-4-phenylbutane (12.02g, 40.4 mmol) was treated with excess isoamylamine as described previously to give 12.06g, 78% of N[3(S)-benzyloxycarbonyl-amino-2(R)-hydroxy-4-phenylbutyl]N-isoamyl amine, mp 130-132°C, MH⁺ m/z = 385.
Part B:
   The product from Part A (470.6mg, 1.22 mmol) was treated with one equivalent of tert-butyl isocyanate as described previously to give 550mg, 93% of (2R,3S)-3(N-benzyloxycarbonyl)amido-1-isoamyl-1-(tert-butylcarbamoyl)amino-4-phenyl-2-butanol as a foam, MH⁺, m/z =484.
Part C:
   The product from Part B (500mg, 1.03 mmol) was hydrogenated in the presence of 10% palladium-on-carbon catalyst to give 356mg, 99%, of (2R, 3S)-3-amino-1-isoamyl-1-(tert-butyl-carbamoyl)amino-4-phenyl-2-butanol as an oil.
Part D:
   The product from Part C (197.1mg, 0.564 mmol) in 2mL of tetrahydrofuran was treated with tert-butylisocyanate (55.9mg, 0.564 mmol) *via* syringe. The mixture was allowed to stand at room temperature for 3 hours and then concentrated *in vacuo* to give a white foam 240mg, 95%, of (2R, 3S)-3(N-tert-butylcarbamoyl)amino-1-isoamyl-1(tert-butylcarbamoyl)amino-4-phenyl-2-butanol,mp 79-81°C, MH⁺ m/z = 449.

### EXAMPLE 6

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Table 6. The enzyme method is described below. The substrate is 2-aminobenzoyl-Ile-Nle-Phe(p-NO₂)-Gln-ArgNH₂. The positive control is MVT-101 (Miller, M. et al, Science, 246, 1149 (1989)] The assay conditions are as follows:
Assay buffer:
20 mM sodium phosphate, pH 6.4
20% glycerol
1 mM EDTA
1 mM DTT
0.1% CHAPS

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to 10x the test concentration; 10µ1 of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

**TABLE 6**

| Compound | IC₅₀ |
|---|---|
| (2S,3S)-3-(N-1,1-dimethylethoxycarbonyl)amido-1-(L-phenylalanyl-L-leucylcarbamoyl)amino-4-phenyl butanol | 5µM |
| | |
| Carbamic acid, [3-[[[(1,1-dimethylethyl)amino]carbonyl](3-methylbutyl)amino]-2-hydroxy-1-(phenylmethyl)propyl]-, (4-methoxyphenyl)methyl ester, [1S-[1R*,2S*]] | 489nM |
| | |
| Carbamic acid, [3-[[[(1,1-dimethylethyl)amino]carbonyl](3-methylbutyl)amino]-2-hydroxy-1-(phenylmethyl)propyl]-, phenylmethyl ester, [1S-[1R*,2S*]] | 112nM |
| | |
| Butaneamide, N-[3-[[[(1,1-dimethylethyl)amino]carbonyl](3-methylbutyl)amino]-2-hydroxy-1-(phenylmethyl)propyl]-3,3-dimethyl-, [1S-[1R*,2S*]] | 3.7mM |
| | |
| Phenylmethylamide, N-[3-[[[(1,1-dimethylethyl)amino]carbonyl](3-methylbutyl)amino]-2-hydroxy-1-(phenylmethyl)propyl]-3,3-dimethyl-, [1S-[1R*,2S*]] | 850nM |

### Example 7

The effectiveness of the compounds listed in Table 7 were determined in the above-described enzyme assay and in a CEM cell assay.

The HIV inhibition assay method of acutely infected cells is an automated tetrazolium based colorimetric assay essentially that reported by Pauwles et al, J. Virol. Methods 20, 309-321 (1988). Assays were performed in 96-well tissue culture plates. CEM cells, a CD4⁺ cell line, were grown in RPMI-1640 medium (Gibco) supplemented with a 10% fetal calf serum and were then treated with polybrene (2µg/ml). An 80 µl volume of medium containing 1 x 10⁴ cells was dispensed into each well of the tissue culture plate. To each well was added a 100µl volume of test compound dissolved in tissue culture medium (or medium without test compound as a control) to achieve the desired final concentration and the cells were incubated at 37°C for 1 hour. A frozen culture of HIV-1 was diluted in culture medium to a concentration of 5 x 10⁴ TCID₅₀ per ml (TCID₅₀ = the dose of virus that infects 50% of cells in tissue culture), and a 20µL volume of the virus sample (containing 1000 TCID₅₀ of virus) was added to wells containing test compound and to wells containing only medium (infected control cells). Several wells received culture medium without virus (uninfected control cells). Likewise, the intrinsic toxicity of the test compound was determined by adding medium without virus to several wells containing test compound. In summary, the tissue culture plates contained the following experiments:

| | Cells | Drug | Virus |
|---|---|---|---|
| 1. | + | - | - |
| 2. | + | + | - |
| 3. | + | - | + |
| 4. | + | + | + |

In experiments 2 and 4 the final concentrations of test compounds were 1, 10, 100 and 500 µg/ml. Either azidothymidine (AZT) or dideoxyinosine (ddI) was included as a positive drug control. Test compounds were dissolved in DMSO and diluted into tissue culture medium so that the final DMSO concentration did not exceed 1.5% in any case. DMSO was added to all control wells at an appropriate concentration.

Following the addition of virus, cells were incubated at 37°C in a humidified, 5% CO₂ atmosphere for 7 days. Test compounds could be added on days 0, 2 and 5 if desired. On day 7, post-infection, the cells in each well were resuspended and a 100µl sample of each cell suspension was removed for assay. A 20µL volume of a 5 mg/ml solution of 3-(4,5-dimethylthiazol-2-yl)-2,5-diphenyltetrazolium bromide (MTT) was added to each 100µL cell suspension, and the cells were incubated for 4 hours at 27°C in a 5% CO₂ environment. During this incubation, MTT is metabolically reduced by living cells resulting in the production in the cell of a colored formazan product. To each sample was added 100µl of 10% sodium dodecylsulfate in 0.01 N HCl to lyse the cells, and samples were incubated overnight. The absorbance at 590 nm was determined for each sample using a Molecular Devices microplate reader. Absorbance values for each set of wells is compared to assess viral control infection, uninfected control cell response as well as test compound by cytotoxicity and antiviral efficacy.

**TABLE 7**

| Compound | IC₅₀ | EC₅₀ | TD₅₀ |
|---|---|---|---|
| (2S,3S)-3-(N-1,1-dimethylethoxycarbonyl)amido-1-(L-phenylalanyl-L-leucylcarbamoyl)amino-4-phenyl butanol | 5µM | 13µM | 54µM |
| | | | |
| Carbamic acid, [3-[[[(1,1-dimethylethyl)amino]carbonyl](3-methylbutyl)amino]-2-hydroxy-1-(phenylmethyl)propyl]-, (4-methoxyphenyl)methyl ester, [1S-[1R*,25*]] | 489nM | 820nM | |
| | | | |
| Phenylmethylamide, N-[3-[[[(1,1-dimethylethyl)amino]carbonyl] (3-methylbutyl)amino]-2-hydroxy-1-(phenylmethyl)propyl]-3,3-dimethyl-, [1S-[1R*,2S*]] | 850nM | 2mM | |

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen; containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds. Variations and changes which are obvious to one skilled in the art are intended to be within the scope and nature of the invention which are defined in the appended claims.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

## Claims

1. A compound represented by the formula 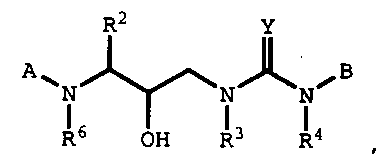 or a pharmaceutically acceptable salt thereof
wherein A is a alkanoyl, aralkanoyl, aroyl, aryloxyalkanoyl, heteroaroyl, alkoxycarbonyl, aralkoxycarbonyl, aryloxycarbonyl, cycloalkylalkoxycarbonyl, cycloalkylcarbonyl, heteroaralkoxycarbonyl, heterocyclylcarbonyl, heterocyclyloxycarbonyl, alkylaminocarbonyl, aralkylaminocarbonyl or dialkylaminoalkanoyl radical;
B is an hydrogen or alkyl radicals or a radical represented by the formula 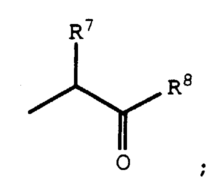
Y is O or S;
R² is an alkyl, aryl, cycloalkyl, cycloalkylalkyl or aralkyl radical, which radical is optionally substituted with -OR⁹, -SR⁹, or halogen radical, wherein R⁹ is a hydrogen or alkyl radical;
R³ is a hydrogen, alkyl, alkenyl, hydroxyalkyl, cycloalkyl, cycloalkylalkyl, heterocycloalkylalkyl, aryl, aralkyl or heteroaralkyl radical;
R⁴ is a hydrogen, alkyl, alkenyl, hydroxyalkyl, cycloalkyl, cycloalkylalkyl, heterocycloalkylalkyl, aryl, aralkyl or heteroaralkyl radical; or R⁴ and B together with the nitrogen atom to which they are bonded form a heterocycloalkyl radical;
R⁶ is a hydrogen or alkyl radical;
R⁷ is an alkyl radical, or the side chain of an amino acid selected from valine, isoleucine, glycine, alanine, allo-isoleucine, asparagine, leucine, glutamine or t-butylglycine; and
R⁸ is an amide derivative of an amino acid; and
wherein alkyl, alone or in combination, is a straight-chain or branched-chain alkyl radical containing from 1 to 8 carbon atoms; cycloalkyl, alone or in combination, is an cyclic alkyl radical containing from 3 to 8 carbon atoms; aryl, alone or in combination, is an unsubstituted phenyl or naphthyl radical, or phenyl or naphthyl radical substituted with an alkyl, alkoxy, halogen, hydroxy or amino radical; heterocycloalkyl, alone or in combination, is a saturated or partially unsaturated monocyclic, bicyclic or tricyclic heterocycle, wherein at least one ring member is a nitrogen, oxygen or sulfur atom and which is unsubstituted or substituted on a substitutable carbon atom with halogen, alkyl, alkoxy or oxo, on a ring secondary nitrogen atom with alkyl, aralkoxycarbonyl, alkanoyl, phenyl or phenylalkyl, or on a ring tertiary nitrogen atom by oxido; and heteroaryl, alone or in combination, is an aromatic monocyclic, bicyclic or tricyclic heterocycloalkyl radical.

2. Compound of Claim 1 wherein A is an alkanoyl, aralkanoyl, alkoxycarbonyl, aralkoxycarbonyl or aryloxycarbonyl radical;
B is a radical represented by the formula 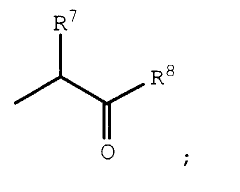
Y is O;
R² is an alkyl, cycloalkyl, cycloalkylalkyl, aryl or aralkyl radical;
R⁴ is a hydrogen or alkyl radical; and
R⁸ is an amide derivative of the amino acid valine, leucine, isoleucine or phenylalanine.

3. Compound of Claim 2 wherein A is an alkoxycarbonyl, aralkoxycarbonyl or aryloxycarbonyl radical;
R² is an alkyl, cycloalkylalkyl or aralkyl radical;
R³ is a hydrogen, alkyl, cycloalkyl, cycloalkylalkyl, heterocycloalkylalkyl, aryl or aralkyl radical;
R⁷ is the side chain of an amino acid selected from valine, isoleucine, asparagine or leucine.

4. Compound of Claim 2 wherein A is an tert-butoxycarbonyl or benzyloxycarbonyl radical;
R² is n-butyl, isobutyl, benzyl, 2-naphthylmethyl or cyclohexylmethyl radical;
R³ is a hydrogen, methyl, cyclohexylmethyl, n-butyl, isobutyl, iso-amyl, neo-pentyl, benzyl, 4-methoxybenzyl, 4-methylbenzyl or 2-naphthylmethyl radical; and
R⁴ is a hydrogen radical.

5. Compound of Claim 1 wherein A is an alkanoyl, aralkanoyl, aroyl, alkoxycarbonyl, aralkoxycarbonyl, cycloalkylcarbonyl of 4-8 ring members, heterocyclylcarbonyl, heteroaralkoxycarbonyl or heteroaroyl radical;
B is an hydrogen or alkyl radical;
R³ is a hydrogen, alkyl, cycloalkyl, cycloalkylalkyl, aryl, aralkyl or heteroaralkyl radical;
R⁴ is a hydrogen, alkyl, cycloalkyl, cycloalkylalkyl, aryl, aralkyl or heteroaralkyl radical; or R⁴ and B together with the nitrogen atom to which they are bonded form a pyrrolidinyl, morpholinyl, piperidinyl or piperazinyl radical;
R⁶ is a hydrogen radical; and
R⁹ is a hydrogen or 1-4 carbon atom alkyl radical.

6. Compound of Claim 5 wherein A is an alkoxycarbonyl, aralkoxycarbonyl, aroyl or heteroaroyl radical;
B is a hydrogen radical;
Y is O;
R² is an alkyl, cycloalkylalkyl or aralkyl radical;
R³ is a alkyl, alkenyl, cycloalkyl or cycloalkylalkyl radical; and
R⁴ is a alkyl, alkenyl, cycloalkyl or cycloalkylalkyl radical.

7. Compound of Claim 5 wherein A is an tert-butoxycarbonyl or benzyloxycarbonyl radical;
B is a hydrogen radical
Y is O;
R² is CH₃SCH₂CH₂-, n-butyl, isobutyl, benzyl, 2-naphthylmethyl or cyclohexylmethyl radical;
R³ is an iso-propyl, iso-butyl, iso-amyl, neo-pentyl, tert-butyl or n-butyl radical; and
R⁴ is an iso-propyl, iso-butyl, iso-amyl, neo-pentyl, tert-butyl or n-butyl radical.

8. Compound of Claim 1 wherein A is a pyrrolidinylcarbonyl, morpholinylcarbonyl, piperidinylcarbonyl or piperazinylcarbonyl radical;
B is a hydrogen or alkyl radical;
R² is an alkyl, aryl, cycloalkylalkyl or aralkyl radical;
R³ is a hydrogen, alkyl, cycloalkyl, cycloalkylalkyl, aryl, aralkyl or heteroaralkyl radical;
R⁴ is a hydrogen, alkyl, cycloalkyl, cycloalkylalkyl, aryl, aralkyl or heteroaralkyl radical; or R⁴ and B together with the nitrogen atom to which they are bonded form a pyrrolidinyl, morpholinyl, piperidinyl or piperazinyl radical; and
R⁶ is a hydrogen radical.

9. Compound of Claim 8 wherein R² is an butyl, isobutyl, benzyl, naphthylmethyl or cyclohexylmethyl radical;
R³ is a hydrogen, methyl, isopropyl, butyl, isobutyl, isoamyl, tert-butyl, neopentyl, cyclohexylmethyl, benzyl, 4-methoxybenzyl, 4-methylbenzyl or 2-naphthylmethyl radical; and
R⁴ is a hydrogen, methyl, isopropyl, butyl, isobutyl, isoamyl, tert-butyl, neopentyl, cyclohexylmethyl, benzyl, 4-methoxybenzyl, 4-methylbenzyl or 2-naphthylmethyl radical.

10. Compound of Claim 1 which is 3(S)-[N-(1,1-dimethylethoxycarbonyl)amino]-1-(L-phenylalanyl-L-leucylcarbonyl)amino-4-phenyl-2(S)-butanol;
1-[N¹-[N²-(1,1-dimethylethyl)amino]carbonyl-N¹-(3-methylbutyl)]amino-3(S)-(N³-(4-methoxyphenyl) methoxycarbonyl) amino-4-phenylbutan-2(R)-ol;
1-[N¹-[N²-(1,1-dimethylethyl)amino]carbonyl-N¹-(2-methylpropyl)]amino-3(S)-(N³-phenylmethoxycarbonyl)amino-4-phenylbutan-2(R)-ol;
1-[N¹-[N²-(1,1-dimethylethyl)amino]carbonyl-N¹-(2-methylpropyl)]amino-3(S)-(N³-1,1-dimethylethoxycarbonyl) amino-4-phenylbutan-2(R)-ol;
1-[N¹-[N²-(1,1-dimethylethyl)amino]carbonyl-N¹-(3-methylbutyl)]amino-3(S)-(N³-phenylmethoxycarbonyl)amino-4-phenylbutan-2(R)-ol;
1-[N¹-[N²-(1,1-dimethylethyl)amino]carbonyl-N¹-(3-methylbutyl)]amino-3(S)-(N³-1,1-dimethylethoxycarbonyl) amino-4-phenylbutan-2(R)-ol;
1-[N¹-(N²,N²-dimethylamino)carbonyl-N¹-(3-methylbutyl)]amino-3(S)-(N³-phenylmethoxycarbonyl)amino-4-phenylbutan-2(R)-ol; or
1-[N¹-(N²,N²-diethylamino)carbonyl-N¹-(3-methylbutyl)]amino-3(S)-(N³-phenylmethoxycarbonyl)amino-4-phenylbutan-2(R)-ol.

11. A pharmaceutical composition comprising a compound of Claim 1 and a pharmaceutically acceptable carrier.

12. A pharmaceutical composition comprising a compound of Claims 2, 5 or 7 and a pharmaceutically acceptable carrier.

13. Use of a compound according to any of claims 1 to 12 for the manufacture of a medicament.

14. Use according to claim 13 of a compound defined in any of claims 1 to 12 for the manufacture of a medicament for inhibiting retroviral protease.

15. Use according to claim 14 wherein the retroviral protease is HIV protease.

16. Use according to claim 13 of a compound defined in any of claims 1 to 12 for the manufacture of a medicament for treatment of a retroviral infection.

17. Use according to claim 16 wherein the retroviral infection is an HIV infection.

18. Use according to claim 13 of a compound defined in any of claims 1 to 12 for the manufacture of a medicament for the treatment of AIDS.

## Patentansprüche

1. Verbindung der Formel 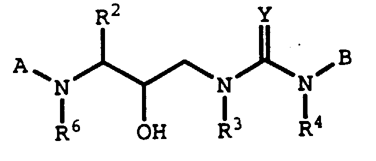 oder ein pharmazeutisch annehmbares Salz hievon, worin A einen Alkanoyl-, Aralkanoyl-, Aroyl-, Aryloxyalkanoyl-, Heteroaroyl-, Alkoxycarbonyl-, Aralkoxycarbonyl-, Aryloxycarbonyl-, Cycloalkylalkoxycarbonyl-, Cycloalkylcarbonyl-, Heteroaralkoxycarbonyl-, Heterocyclylcarbonyl-, Heterocyclyloxycarbonyl-, Alkylaminocarbonyl-, Aralkylaminocarbonyl- oder Dialkylaminoalkanoylrest bedeutet; B einen Wasserstoff- oder Alkylrest darstellt oder einen Rest der Formel 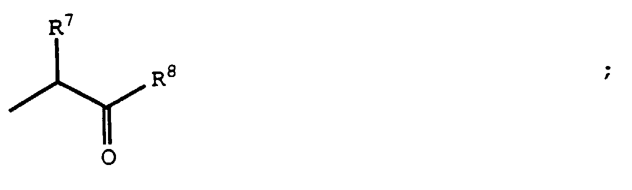 Y die Bedeutung O oder S hat; R² einen Alkyl-, Aryl-, Cycloalkyl-, Cycloalkylalkyl- oder Aralkylrest darstellt, welcher Rest gegebenenfalls substituiert ist mit einem -OR⁹-, -SR⁹- oder Halogenrest, wobei R⁹ einen Wasserstoff- oder Alkylrest bedeutet; R³ einen Wasserstoff-, Alkyl-, Alkenyl-, Hydroxyalkyl-, Cycloalkyl-, Cycloalkylalkyl-, Heterocycloalkylalkyl-, Aryl-, Aralkyl- oder Heteroalkylrest darstellt; R⁴ ein Wasserstoff-, Alkyl-, Alkenyl-, Hydroxyalkyl-, Cycloalkyl-, Cycloalkylalkyl-, Heterocycloalkylalkyl-, Aryl-, Aralkyl- oder Heteroaralkylrest ist; oder R⁴ und B zusammen mit dem Stickstoffatom, an das sie gebunden sind, einen Heterocycloalkylrest bilden; R⁶ einen Wasserstoff- oder Alkylrest bedeutet; R⁷ ein Alkylrest, oder die Seitenkette einer Aminosäure, ausgewählt aus Valin, Isoleucin, Glycin, Alanin, Alloisoleucin, Asparagin, Leucin, Glutamin oder tert.Butylglycin, ist; und R⁸ ein Amid-Derivat einer Aminosäure darstellt; und worin Alkyl, allein oder in Kombination, ein geradkettiger oder verzweigtkettiger Alkylrest mit 1 bis 8 Kohlenstoffatomen ist; Cycloalkyl, allein oder in Kombination, einen cyclischen Alkylrest mit 3 bis 8 Kohlenstoffatomen bedeutet; Aryl, allein oder in Kombination, einen unsubstituierten Phenyl- oder Naphthylrest, oder Phenyl- oder Napthylrest substituiert mit einem Alkyl-, Alkoxy-, Halogen, Hydroxy- oder Aminorest, darstellt; Heterocycloalkyl, allein oder in Kombination, einen gesättigten oder teilweise ungesättigten monocyclischen, bicyclischen oder tricyclischen Heterocyclus bedeutet, wobei zumindest ein Ringglied ein Stickstoff-, Sauerstoff- oder Schwefelatom ist, und welcher unsubstituiert oder substituiert ist an einem substituierbaren Kohlenstoffatom mit Halogen, Alkyl, Alkoxy oder Oxo, an einem sekundären Stickstoff-Ringatom mit Alkyl, Aralkoxycarbonyl, Alkanoyl, Phenyl oder Phenylalkyl, oder an einem tertiären Stickstoff-Ringatom durch Oxido; und Heteroaryl, allein oder in Kombination, einen aromatischen monocyclischen, bicyclischen oder tricyclischen Heterocycloalkylrest bedeutet.

2. Verbindung nach Anspruch 1, worin A einen Alkanoyl-, Aralkanoyl-, Alkoxycarbonyl-, Aralkoxycarbonyl- oder Aryloxycarbonylrest bedeutet; B einen Rest darstellt der Formel 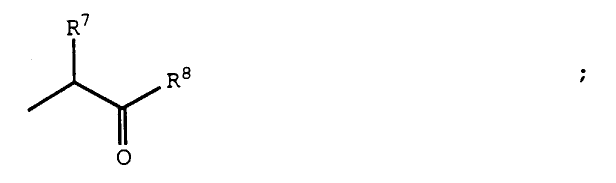 Y die Bedeutung O hat; R² einen Alkyl-, Cycloalkyl-, Cycloalkylalkyl-, Aryl- oder Aralkylrest darstellt; R⁴ ein Wasserstoff- oder Alkylrest ist; und R⁸ ein Amid-Derivat der Aminosäure Leucin, Isoleucin oder Phenylalanin ist.

3. Verbindung nach Anspruch 2, worin A einen Alkoxycarbonyl-, Aralkoxycarbonyl- oder Aryloxycarbonylrest bedeutet; R² einen Alkyl-, Cycloalkylalkyl- oder Aralkylrest darstellt; R³ einen Wasserstoff-, Alkyl-, Cycloalkyl-, Cycloalkylalkyl-, Heterocycloalkylalkyl-, Aryl- oder Aralkylrest darstellt; R⁷ die Seitenkette einer Aminosäure, ausgewählt aus Valin, Isoleucin, Asparagin oder Leucin, bedeutet.

4. Verbindung nach Anspruch 2, worin A einen tert.Butoxycabonyl oder Benzyloxycarbonylrest bedeutet; R² einen n-Butyl-, Isobutyl-, Benzyl-, 2-Naphthylmethyl- oder Cyclohexylmethylrest darstellt; R³ ein Wasserstoff-, Methyl-, Cyclohexylmethyl-, n-Butyl-, Isobutyl-, Isoamyl-, Neopentyl-, Benzyl-, 4-Methoxybenzyl-, 4-Methylbenzyl- oder 2-Naphthylmethylrest ist; und R⁴ einen Wasserstoffrest bedeutet.

5. Verbindung nach Anspruch 1, worin A einen Alkanoyl-, Aralkanoyl-, Aroyl-, Alkoxycarbonyl-, Aralkoxycarbonyl-, 4- bis 8-gliedrigen Cycloalkylcarbonyl-, Heterocyclylcarbonyl-, Heteroaralkoxycarbonyl- oder Heteroaroylrest bedeutet; B einen Wasserstoff- oder Alkylrest darstellt; R³ ein Wasserstoff-, Alkyl-, Cycloalkyl-, Cycloalkylalkyl-, Aryl-, Aralkyl- oder Heteroaralkylrest ist; R⁴ einen Wasserstoff-, Alkyl-, Cycloalkyl-, Cycloalkylalkyl-, Aryl-, Aralkyl- oder Heteroaralkylrest bedeutet; oder R⁴ und B zusammen mit dem Stickstoffatom, an das sie gebunden sind, einen Pyrrolidinyl-, Morpholinyl-, Piperidinyl- oder Piperazinylrest bilden; R⁶ einen Wasserstoffrest darstellt; und R⁹ ein Wasserstoff- oder Alkylrest mit 1 bis 4 Kohlenstoffatomen ist.

6. Verbindung nach Anspruch 5, worin A einen Alkoxycarbonyl-, Aralkoxycarbonyl-, Aroyl- oder Heteroaroylrest bedeutet; B einen Wasserstoffrest darstellt; Y die Bedeutung O hat; R² ein Alkyl-, Cycloalkylalkyl- oder Aralkylrest ist; R³ einen Alkyl-, Alkenyl-, Cycloalkyl- oder Cycloalkylalkylrest darstellt; und R⁴ einen Alkyl-, Alkenyl-, Cycloalkyl- oder Cycloalkylalkylrest bedeutet.

7. Verbindung nach Anspruch 5, worin A einen tert.Butoxycabonyl oder Benzyloxycarbonylrest bedeutet; B einen Wasserstoffrest darstellt; Y die Bedeutung O hat; R² ein CH₃SCH₂CH₂-, n-Butyl-, Isobutyl-, Benzyl-, 2-Naphthylmethyl- oder Cyclohexylmethylrest ist; R³ einen Isopropyl-, Isobutyl-, Isoamyl-, Neopentyl-, tert.Butyl- oder n-Butylrest darstellt; und R⁴ einen Isopropyl-, Isobutyl-, Isoamyl-, Neopentyl-, tert.Butyl- oder n-Butylrest bedeutet.

8. Verbindung nach Anspruch 1, worin A einen Pyrrolidinylcarbonyl-, Morpholinylcarbonyl-, Piperidinylcarbonyl- oder Piperazinylcarbonylrest bedeutet; B einen Wasserstoff- oder Alkylrest darstellt; R² ein Alkyl-, Aryl-, Cycloalkylalkyl- oder Aralkylrest ist; R³ einen Wasserstoff-, Alkyl-, Cycloalkyl-, Cycloalkylalkyl-, Aryl-, Aralkyl- oder Heteroaralkylrest bedeutet; R⁴ einen Wasserstoff-, Alkyl-, Cycloalkyl-, Cycloalkylalkyl-, Aryl-, Aralkyl- oder Heteroaralkylrest darstellt; oder R⁴ und B zusammen mit dem Stickstoffatom, an das sie gebunden sind, einen Pyrrolidinyl-, Morpholinyl-, Piperidinyl- oder Piperazinylrest bilden; und R⁶ ein Wasserstoffrest ist.

9. Verbindung nach Anspruch 8, worin R² einen Butyl-, Isobutyl-, Benzyl-, Naphthylmethyl- oder Cyclohexylmethylrest bedeutet; R³ einen Wasserstoff-, Methyl-, Isopropyl-, Butyl-, Isobutyl-, Isoamyl-, tert.Butyl-, Neopentyl-, Cyclohexylmethyl-, Benzyl-, 4-Methoxybenzyl-, 4-Methylbenzyl- oder 2-Naphthylmethylrest darstellt; und R⁴ ein Wasserstoff-, Methyl-, Isopropyl-, Butyl-, Isobutyl-, Isoamyl-, tert.Butyl-, Neopentyl-, Cyclohexylmethyl-, Benzyl-, 4-Methoxybenzyl-, 4-Methylbenzyl- oder 2-Naphthylmethylrest ist.

10. Verbindung nach Anspruch 1, nämlich:
3(S)-[N-(1,1-Dimethylethoxycarbonyl)-amino]-1-(L-phenylalanyl-L-leucylcarbonyl)-amino-4-phenyl-2(S)-butanol;
1-[N¹-[N²-(1,1-Dimethylethyl)-amino]-carbonyl-N¹-(3-methylbutyl)]-amino-3(S)-(N³-(4-methoxyphenyl)-methoxycarbonyl)-amino-4-phenylbutan-2(R)-ol;
1-[N¹-[N²-(1,1-Dimethylethyl)-amino]-carbonyl-N¹-(2-methylpropyl)]-amino-3(S)-(N³-phenylmethoxycarbonyl)-amino-4-phenylbutan-2(R)-ol;
1-[N¹-[N²-(1,1-Dimethylethyl)-amino]-carbonyl-N¹-(2-methylpropyl)]-amino-3(S)-(N³-1,1-dimethylethoxycarbonyl)-amino-4-phenylbutan-2(R)-ol;
1-[N¹-[N²-(1,1-Dimethylethyl)-amino]-carbonyl-N¹-(3-methylbutyl)]-amino-3(S)-(N³-phenylmethoxycarbonyl)-amino-4-phenylbutan-2(R)-ol;
1-[N¹-[N²-(1,1-Dimethylethyl)-amino]-carbonyl-N¹-(3-methylbutyl)]-amino-3(S)-(N³-1,1-dimethylethoxycarbonyl)-amino-4-phenylbutan-2(R)-ol;
1-[N¹-(N²,N²-Dimethylamino)-carbonyl-N¹-(3-methylbutyl)]amino-3(S)-(N³-phenylmethoxycarbonyl)-amino-4-phenylbutan-2(R)-ol; oder
1-[N¹-(N²,N²-Diethylamino)-carbonyl-N¹-(3-methylbutyl)]amino-3(S)-(N³-phenylmethoxycarbonyl)-amino-4-phenylbutan-2(R)-ol.

11. Pharmazeutische Zusammensetzung, welche eine Verbindung nach Anspruch 1 und einen pharmazeutisch annehmbaren Träger umfaßt.

12. Pharmazeutische Zusammensetzung, welche eine Verbindung nach Anspruch 2, 5 oder 7 und einen pharmazeutisch annehmbaren Träger umfaßt.

13. Verwendung einer Verbindung nach einem der Ansprüche 1 bis 12 bei der Herstellung eines Medikaments.

14. Verwendung nach Anspruch 13 einer in einem der Ansprüche 1 bis 12 definierten Verbindung bei der Herstellung eines Medikaments zur Inhibierung retroviraler Protease.

15. Verwendung nach Anspruch 14, wobei die retrovirale Protease HIV-Protease ist.

16. Verwendung nach Anspruch 13 einer in einem der Ansprüche 1 bis 12 definierten Verbindung bei der Herstellung eines Medikaments zur Behandlung einer Retrovirusinfektion.

17. Verwendung nach Anspruch 16, wobei die Retrovirusinfektion eine HIV-Infektion ist.

18. Verwendung nach Anspruch 13 einer in einem der Ansprüche 1 bis 12 definierten Verbindung bei der Herstellung eines Medikaments zur Behandlung von AIDS.

## Revendications

1. Composé répondant à la formule : 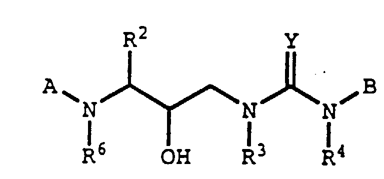 ou sel pharmaceutiquement acceptable de ce dernier,
dans laquelle A représente un radical alcanoyle, aralcanoyle, aroyle, aryloxyalcanoyle, hétéroaroyle, alcoxycarbonyle, aralcoxycarbonyle, aryloxycarbonyle, cycloalkylalcoxycarbonyle, cycloalkylcarbonyle, hétéroaralcoxycarbonyle, hétérocyclylcarbonyle, hétérocyclyloxycarbonyle, alkylaminocarbonyle, aralkylaminocarbonyle ou dialkylaminoalcanoyle ;
B représente un atome d'hydrogène, un radical alkyle ou un radical répondant à la formule : 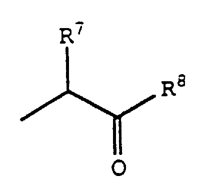
Y représente O ou S,
R² représente un radical alkyle, aryle, cycloalkyle, cycloalkylalkyle ou aralkyle, lequel radical est éventuellement substitué par -OR⁹, -SR⁹, ou un radical halogéno, sachant que R⁹ représente un atome d'hydrogène ou un radical alkyle ;
R³ représente un atome d'hydrogène, un radical alkyle, alcényle, hydroxyalkyle, cycloalkyle, cycloalkylalkyle, hétérocycloalkylalkyle, aryle, aralkyle ou hétéroaralkyle ;
R⁴ représente un atome d'hydrogène, un radical alkyle, alcényle, hydroxyalkyle, cycloalkyle, cycloalkylalkyle, hétérocycloalkylalkyle, aryle, aralkyle ou hétéroaralkyle ; ou R⁴ et B forment ensemble avec l'atome d'azote auxquels ils sont reliés un radical hétérocycloalkyle ;
R⁶ représente un atome d'hydrogène ou un radical alkyle ;
R⁷ représente un radical alkyle, ou la chaîne secondaire d'un acide aminé choisi parmi la valine, l'isoleucine, la glycine, l'alanine, l'alloisoleucine, l'asparagine, la leucine, la glutamine ou la t-butylglycine ; et
R⁸ représente un dérivé d'amide d'un acide aminé ; et
dans laquelle le radical alkyle, seul ou en combinaison, est un radical alkyle à chaîne droite ou ramifiée, comportant de 1 à 8 atomes de carbone ; le radical cycloalkyle, seul ou en combinaison, est un radical alkyle cyclique comportant de 3 à 8 atomes de carbone ; le radical aryle, seul ou en combinaison, est un radical phényle ou naphtyle non substitué, ou un radical phényle ou naphtyle substitué par un radical alkyle, alcoxy, halogéno, hydroxy ou amino ; le radical hétérocycloalkyle, seul ou en combinaison, est un hétérocycle monocyclique, bicyclique ou tricyclique, saturé ou partiellement insaturé, dans lequel au moins un chaînon du cycle est un atome d'azote, d'oxygène ou de soufre et qui n'est pas substitué ou qui est substitué sur un atome de carbone, pouvant porter un substituant, par un atome d'halogène, un radical alkyle, alcoxy ou oxo, sur un atome d'azote secondaire du cycle par un radical alkyle, aralcoxycarbonyle, alcanoyle, phényle ou phénylalkyle, ou sur un atome d'azote tertiaire du cycle par un radical oxydo; et le radical hétéroaryle, seul ou en combinaison, est un radical hétérocycloalkyle aromatique monocyclique, bicyclique ou tricyclique.

2. Composé conforme à la revendication 1, dans lequel A représente un groupe alcanoyle, aralcanoyle, alcoxycarbonyle, aralcoxycarbonyle ou aryloxycarbonyle ;
B représente un radical répondant à la formule : 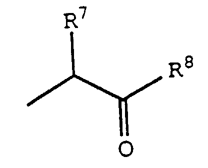
Y représente O ;
R² représente un radical alkyle, cycloalkyle, cycloalkylalkyle, aryle ou aralkyle ;
R⁴ représente un atome d'hydrogène ou un radical alkyle ; et
R⁸ représente le dérivé amide de l'acide aminé valine, leucine, isoleucine ou phénylalanine.

3. Composé conforme à la revendication 2, dans lequel A représente un radical alcoxycarbonyle, aralcoxycarbonyle ou aryloxycarbonyle ;
R² représente un radical alkyle, cycloalkylalkyle ou aralkyle ;
R³ représente un atome d'hydrogène, un radical alkyle, cycloalkyle, cycloalkylalkyle, hétérocycloalkylalkyle, aryle ou aralkyle ;
R⁷ représente la chaîne secondaire d'un acide aminé choisi parmi la valine, l'isoleucine, l'asparagine ou la leucine.

4. Composé conforme à la revendication 2, dans lequel A représente un radical tert-butoxycarbonyle ou benzyloxycarbonyle ;
R² représente un radical n-butyle, isobutyle, benzyle, 2-naphtylméthyle ou cyclohexylméthyle ;
R³ représente un atome d'hydrogène, un radical méthyle, cyclohexylméthyle, n-butyle, isobutyle, iso-amyle, néopentyle, benzyle, 4-méthoxybenzyle, 4-méthylbenzyle ou 2-naphtylméthyle ; et
R⁴ représente un atome d'hydrogène.

5. Composé conforme à la revendication 1, dans lequel A représente un radical alcanoyle, aralcanoyle, aroyle, alcoxycarbonyle, aralcoxycarbonyle, cycloalkylcarbonyle comportant de 4 à 8 chaînons dans le cycle, hétérocyclylcarbonyle, hétéroaralcoxycarbonyle ou hétéroaroyle ;
B représente un atome d'hydrogène ou un radical alkyle ;
R³ représente un atome d'hydrogène, un radical alkyle, cycloalkyle, cycloalkylalkyle, aryle, aralkyle ou hétéroaralkyle ;
R⁴ représente un atome d'hydrogène, un radical alkyle, cycloalkyle, cycloalkylalkyle, aryle, aralkyle ou hétéroaralkyle ; ou R⁴ et B forment ensemble avec l'atome d'azote auxquels ils sont reliés un radical pyrrodinyle, morpholinyle, pipéridinyle ou pipérazinyle ;
R⁶ représente un atome d'hydrogène ; et
R⁹ représente un atome d'hydrogène ou un radical alkyle comportant de 1 à 4 atomes de carbone.

6. Composé conforme à la revendication 5, dans lequel A représente un radical alcoxycarbonyle, aralcoxycarbonyle, aroyle ou hétéroaroyle ;
B représente un atome d'hydrogène ;
Y représente O ;
R² représente un radical alkyle, cycloalkylalkyle ou aralkyle ;
R³ représente un groupe alkyle, alcényle, cycloalkyle ou cycloalkylalkyle ; et
R⁴ représente un groupe alkyle, alcényle, cycloalkyle ou cycloalkylalkyle.

7. Composé conforme à la revendication 5, dans lequel A représente un radical tert-butoxycarbonyle ou benzyloxycarbonyle ;
B représente un atome d'hydrogène ;
Y représente O ;
R² représente CH₃SCH₂CH₂-, n-butyle, isobutyle, benzyle, 2-naphtylméthyle ou cyclohexylméthyle ;
R³ représente un radical isopropyle, isobutyle, iso-amyle, néopentyle, tert-butyle ou n-butyle ; et
R⁴ représente un radical isopropyle, isobutyle, iso-amyle, néopentyle, tert-butyle ou n-butyle.

8. Composé conforme à la revendication 1, dans lequel A représente un radical pyrrolidinylcarbonyle, morpholinylcarbonyle, pipéridinylcarbonyle ou pipérazinylcarbonyle ;
B représente un atome d'hydrogène ou un radical alkyle ;
R² représente un groupe alkyle, aryle, cycloalkylalkyle ou aralkyle ;
R³ représente un atome d'hydrogène ou un radical alkyle, cycloalkyle, cycloalkylalkyle, aryle, aralkyle ou hétéroaralkyle ;
R⁴ représente un atome d'hydrogène, un groupe alkyle, cycloalkyle, cycloalkylalkyle, aryle, aralkyle ou hétéroaralkyle ; ou R⁴ et B forment ensemble avec l'atome d'azote auxquels ils sont reliés, un radical pyrrolidinyle, morpholinyle, pipéridinyle ou pipérazinyle ; et R⁶ représente un atome d'hydrogène.

9. Composé conforme à la revendication 8, dans lequel R² représente un radical butyle, isobutyle, benzyle, naphtylméthyle ou cyclohexylméthyle ;
R³ représente un atome d'hydrogène, un radical méthyle, isopropyle, butyle, isobutyle, isoamyle, tert-butyle, néopentyle, cyclohexylméthyle, benzyle, 4-méthoxybenzyle, 4-méthylbenzyle ou 2-naphtylméthyle ; et
R⁴ représente un atome d'hydrogène, un radical méthyle, isopropyle, butyle, isobutyle, isoamyle, tert-butyle, néopentyle, cyclohexylméthyle, benzyle, 4-méthoxybenzyle, 4-méthylbenzyle, ou 2-naphtylméthyle.

10. Composé conforme à la revendication 1, qui est le 3(S)[N-(1,1-diméthyléthoxycarbonyl)amino]-1-(L-phénylalanyl-L-leucylcarbonyl)amino-4-phényl-2(S)-butanol; le 1-[N¹-[N²-(1,1-diméthyléthyl)amino]carbonyl-N¹-(3-méthylbutyl)]amino-3(S)-(N³-(4-méthoxyphényl)méthoxycarbonyl)amino-4-phénylbutan-2(R)-ol; le 1-[N¹-[N²-(1,1-diméthyléthyl)amino]carbonyl-N¹-(2-métylpropyl)] amino-3(S)-(N³-phénylméthoxycarbonyl)amino-4-phénylbutan-2(R)-ol; le 1-[N¹-[N²-(1,1-diméthyléthyl)amino]carbonyl-N¹-(2-méthylpropyl)]amino-3(S)-(N³-1,1-diméthyléthoxycarbonyl)amino-4-phénylbutan-2(R)-ol; le 1-[N¹-[N²-(1,1-diméthyléthyl)amino]carbonyl-N¹-(3-méthylbutyl)]amino-3(S)-(N³-phénylméthoxycarbonyl)amino-4-phénylbutan-2(R)-ol; le (N¹[N²-(1,1-diméthyléthyl)amino]carbonyl-N¹-(3-méthylbutyl)]amino-3(S)-(N³-1,1-diméthyléthoxycarbonyl)amino-4-phénylbutan-2(R)-ol; le 5 1-[N¹-(N²,N²-diméthylamino)carbonyl-N¹-(3-méthylbutyl)]amino-3(S)-(N³-phénylméthoxycarbonyl)amino-4-phénylbutan2(R)-ol; ou le 1-[N¹-(N²,N²-diéthylamino)carbonyl-N¹-(3-méthylbutyl)] amino-3(S)-(N³-phénylméthoxycarbonyl)amino-4-phénylbutan-2(R)-ol.

11. Composition pharmaceutique comprenant un composé conforme à la revendication 1 et un porteur pharmaceutiquement acceptable.

12. Composition pharmaceutique comprenant un composé conforme aux revendications 2, 5 ou 7 et un porteur pharmaceutiquement acceptable.

13. Utilisation d'un composé conforme aux revendications 1 à 12 pour la fabrication d'un principe actif.

14. Utilisation conforme à la revendication 13, d'un composé défini dans l'une quelconque des revendications 1 à 12, pour la fabrication d'un principe actif destiné à inhiber la protéase rétrovirale.

15. Utilisation conforme à la revendication 14, dans laquelle la protéase rétrovirale est la protéase de VIH.

16. Utilisation conforme à la revendication 13, d'un composé défini dans l'une quelconque des revendications 1 à 12, pour la fabrication d'un principe actif destiné à traiter une infection par un rétrovirus.

17. Utilisation conforme à la revendication 16, dans laquelle l'infection par un rétrovirus est une infection par le VIH.

18. Utilisation conforme à la revendication 13, d'un composé défini dans l'une quelconque des revendications 1 à 12, pour la fabrication d'un principe actif destiné à traiter le SIDA.
